(19) Europäisches Patentamt
European Patent Office
Office européen des brevets

(11) Veröffentlichungsnummer: **0 122 426 B1**

(12) **EUROPÄISCHE PATENTSCHRIFT**

(45) Veröffentlichungstag der Patentschrift: **27.01.88**

(51) Int. Cl.⁴: **C 07 D 311/72**, C 07 D 311/58

(21) Anmeldenummer: **84102408.6**

(22) Anmeldetag: **07.03.84**

(54) **Neue optisch aktive Chromanderivate, Verfahren zu deren Herstellung sowie neue Zwischenprodukte.**

(30) Priorität: **15.03.83 DE 3309159**

(43) Veröffentlichungstag der Anmeldung: **24.10.84 Patentblatt 84/43**

(45) Bekanntmachung des Hinweises auf die Patenterteilung: **27.01.88 Patentblatt 88/4**

(84) Benannte Vertragsstaaten: **CH DE FR GB LI NL**

(56) Entgegenhaltungen:
**EP - A - 0 036 160**
**EP - A - 0 065 368**

**JOURNAL OF THE AMERICAN CHEMICAL SOCIETY, Band 101, Nr. 22, 24. Oktober 1979, Seiten 6710-6716, Washington, US; N. COHEN u.a.: "A novel total synthesis of (2R,4'R,8'R)-alpha-tocopherol (vitamin E). Construction of chiral chromans from an optically active, nonaromatic precursor"**
**HELVETICA CHIMICA ACTA, Band 61, Fasz. 2, Nr. 73, 1978, Seiten 837-843; N. COHEN u.a.: "Studies on the synthesis of (2R,4'R,8'R)-alpha-tocopherol alternative syntheses of 2-chroman-acetic acid intermediates"**

(73) Patentinhaber: **BASF Aktiengesellschaft, Carl-Bosch-Strasse 38, D-6700 Ludwigshafen (DE)**

(72) Erfinder: **Gehrken, Henning-Peter, Dr., Wiesenweg 6, D-6715 Lambsheim (DE)**
Erfinder: **Ernst, Hansgeorg, Dr., Bruesseler Ring 38, D-6700 Ludwigshafen (DE)**
Erfinder: **Paust, Joachim, Dr., Ringstrasse 3, D-6708 Neuhofen (DE)**

**EP 0 122 426 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft ein Verfahren zur Herstellung von neuen optisch aktiven Chromanderivaten der allgemeinen Formeln Ia und Ib

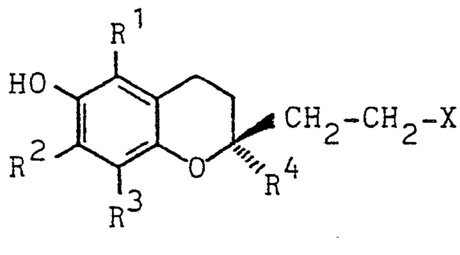

(Ia)

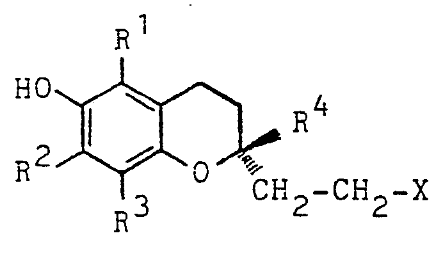

(Ib)

in denen die Reste $R^1$ bis $R^4$ Wasserstoff oder eine Methylgruppe bedeuten und X für -OH, $-O-CO-R^6$, $-O-R^6$-O-Tosyl, O-Mesyl, O-Benzolsulfonyl, Cl, Br oder J stehen, worin $R^6$ eine $C_1$- bis $C_4$-Alkylgruppe bedeutet.

Weiterhin betrifft die Erfindung die besonders interessanten Vertreter dieser neuen Verbindungen, nämlich die optisch aktiven Chromanderivate der Formeln Ia und Ib, in denen $R^1$ bis $R^4$ für Methyl stehen und X für -OH, Cl, Br, J, $-O-CH_3$, -O-tert.-butyl, $-O-CO-CH_3$ oder -O-Tosyl, insbesondere für Br, Cl oder O-Tosyl steht.

Aus der EP-A 0 036 160 ist zwar ein Verfahren zur Herstellung von 2-Hydroxyalkylchromanen bekannt, jedoch wird das Auftreten optischer Isomere hierin nicht erwähnt. Darüber hinaus sind aus J. Am. Chem. Soc. 101 (1979), Seiten 6710-6716, und EP-A 0 065 368 Verfahren zur Herstellung von (S)-(+)-3,4-Dihydro-6-hydroxy-2,5,7,8-tetramethyl-2H-1-benzopyran-2-methanol (was man auch (S)-2,5,7,8-Tetramethyl-6-hydroxy-2-(hydroxy-methyl)-chroman nennen kann) als Zwischenprodukt für die Herstellung von 2R, 4'R, 8'R)-α-Tocopherol bekannt. Abgesehen davon, dass diese Verfahren recht aufwendig sind, ist festzustellen, dass sich dieses 2-Methanol-chroman-Derivat in seinem chemischen Verhalten beträchtlich von dem erfindungsgemässen 2-Ethanol-chroman-Derivat unterscheidet. So sind beispielsweise die in der vorliegenden Anmeldung beschriebenen Halogenierungen mit dem 2-Methanol-chroman-Derivat nicht möglich. Hierdurch bedingt ist die Herstellung von 2R-α-Tocopherolen durch Verknüpfung der erfindungsgemässen 2-Ethanol-chroman-Derivate mit einer geeigneten $C_{14}$-Grignardverbindung einfacher zu realisieren als die entsprechende Umsetzung eines 2-Methanol-chroman-Derivats mit einer geeigneten $C_{15}$-Grignardverbindung.

Weiterhin betrifft die Erfindung diastereomere Ester der allgemeinen Formeln IV und IV' als neue Zwischenprodukte dieses Herstellungsverfahrens.

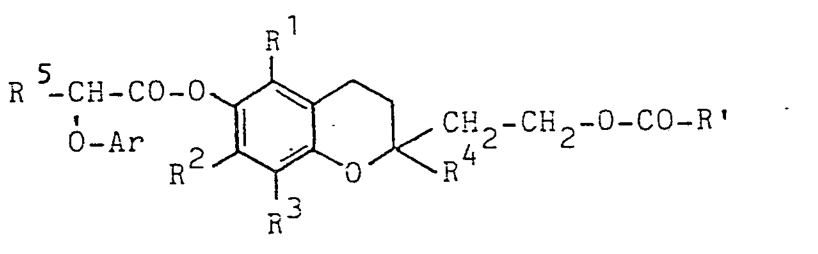

(IV)

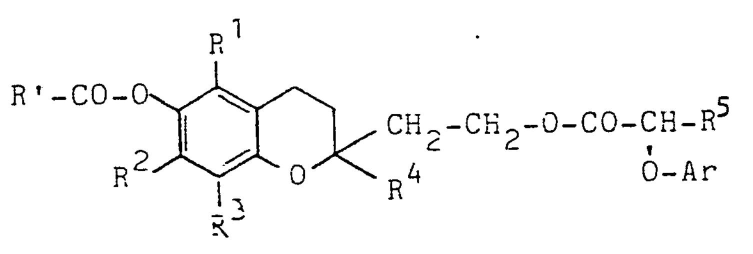

(IV')

in denen $R^5$ eine Methylgruppe, R' eine $C_1$- bis $C_4$-Alkylgruppe und Ar eine Phenyl-, α-Naphthyl- oder β-Naphthylgruppe bedeutet, die ihrerseits durch eine Methylgruppe, Chlor oder Brom substituiert sein kann.

Genannt seien insbesondere als Chromanderivate der Formeln Ia und Ib:

(S)-2,5,7,8-Tetramethyl-6-hydroxy-2-(2-hydroxyethyl)-chroman,

(R)-2,5,7,8-Tetramethyl-6-hydroxy-2-(2-hydroxy-ethyl)-chroman,
(S)-2,5,7,8-Tetramethyl-6-hydroxy-2-(2-brom-ethyl)-chroman,
(R)-2,5,7,8-Tetramethyl-6-hydroxy-2-(2-brom-ethyl)-chroman,
(S)-2,5,7,8-Tetramethyl-6-hydroxy-2-(2-chlor-ethyl)-chroman und
(R)-2,5,7,8-Tetramethyl-6-hydroxy-2-(2-chlor-ethyl)-chroman,

von denen die (S)-Derivate mit einer Abgangsgruppe in der Seitenkette besondere Bedeutung für die Herstellung von (2R, 4'R, 8'R)- und 2R, 4'RS, 8'RS)-α-Tocopherol haben.

Als diastereomere Ester der Formeln IV und IV' seien genannt:

2,5,7,8-Tetramethyl-6-[2'-(o-methyl-p-chlor-phenoxy)-propionyl]-2(-2-acetoxy-ethyl)-chroman;
2,5,7,8-Tetramethyl-6-acetoxy-2-[2-(2'-α-naphthoxy)-propionyl-ethyl]-chroman und
2,5,7,8-Tetramethyl-6-hydroxy-2-[2-(2'-α-naphthoxy)-propionyl-ethyl]-chroman.

Die Substituenten der optisch aktiven Verbindungen der Formeln Ia und Ib sowie der übrigen in dieser Anmeldung benutzten optisch aktiven Strukturformel sind, sofern sie vor der Ebene des Moleküls liegen, durch das Zeichen ▲, sofern sie hinter der Ebene des Moleküls liegen durch das Zeichen ≡ gekennzeichnet. Die Substituenten der stereochemisch nicht besonders gekennzeichneten Strukturformeln können entweder R- oder S-orientiert sein, oder die Verbindung kann als Gemisch der R- und S-Isomeren vorliegen.

Die Chromanderivate der Formeln Ia und Ib, in denen $R^1$ bis $R^4$ für Methyl stehen, und X für eine Abgangsgruppe steht, haben grosse Bedeutung für die Herstellung von α-Tocopherol (Vitamin E) und zwar, abhängig davon, ob sie in der 2 R-, 2 S- oder 2 RS-Form vorliegen, sind sie Schlüsselsubstanzen sowohl für die Herstellung von natürlichem optisch aktivem α-Tocopherol (2R, 4'R, 8'R-α-Tocopherol) als auch von anderen optisch aktiven Isomeren, insbesondere dem ebenfalls biologisch sehr aktiven (2R, 4'RS, 8'RS)-α-Tocopherol.

Aus der DE-OS 2 602 509 ist eine Tocopherolsynthese durch Schlosser-Fouquet-Kupplung eines Chromderivats der Formel

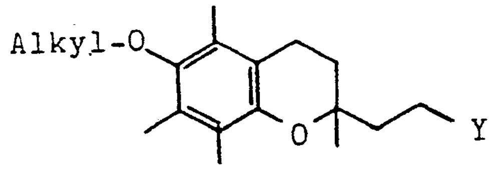

in der y eine austretende Gruppe bedeutet, mit einer geeigneten $C_{14}$-Grignard-Verbindung bekannt. Die Herstellung eines für diese Synthese geeigneten optisch aktiven Chromanbausteins könnte nach einem in der DE-OS 2 364 165 beschriebenen Verfahren erfolgen.

Gemäss diesem Verfahren wird das Chromanderivat der Formel

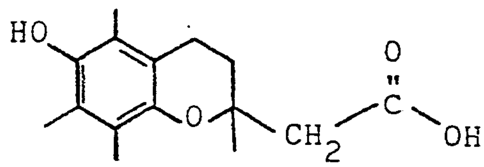

mit Hilfe einer optisch aktiven Base, wie Phenylethylamin über das diastereomere Salzpaar in die optischen Antipoden zerlegt. Die erhaltene optisch aktive Chromanylessigsäure könnte dann nach Veresterung und Schutz der phenolischen Hydroxylgruppe zu dem entsprechenden optisch aktiven Chromanylethanol reduziert werden, aus dem durch Tosylierung ein nach dem Verfahren der DE-OS 2 602 509 kupplungsfähiges Chromanderivat erhalten werden könnte. Insgesamt ergäbe sich unter weiterer Berücksichtigung der Angaben von Cohen et al. in J. Org. Chem. 41 (1976), Seiten 3505 und Scott et al. in Helv. Chim. Acta 59 (1976) Seiten 290 f sowie Cohen et al. in Helvetica Chimica Acta Vol. 61, Fasc. 2 (1978) Nr. 73, Seiten 837–843, insbesondere Seite 839, folgender Reaktionsweg für die Herstellung eines solchen kupplungsfähigen Chromanderivates:

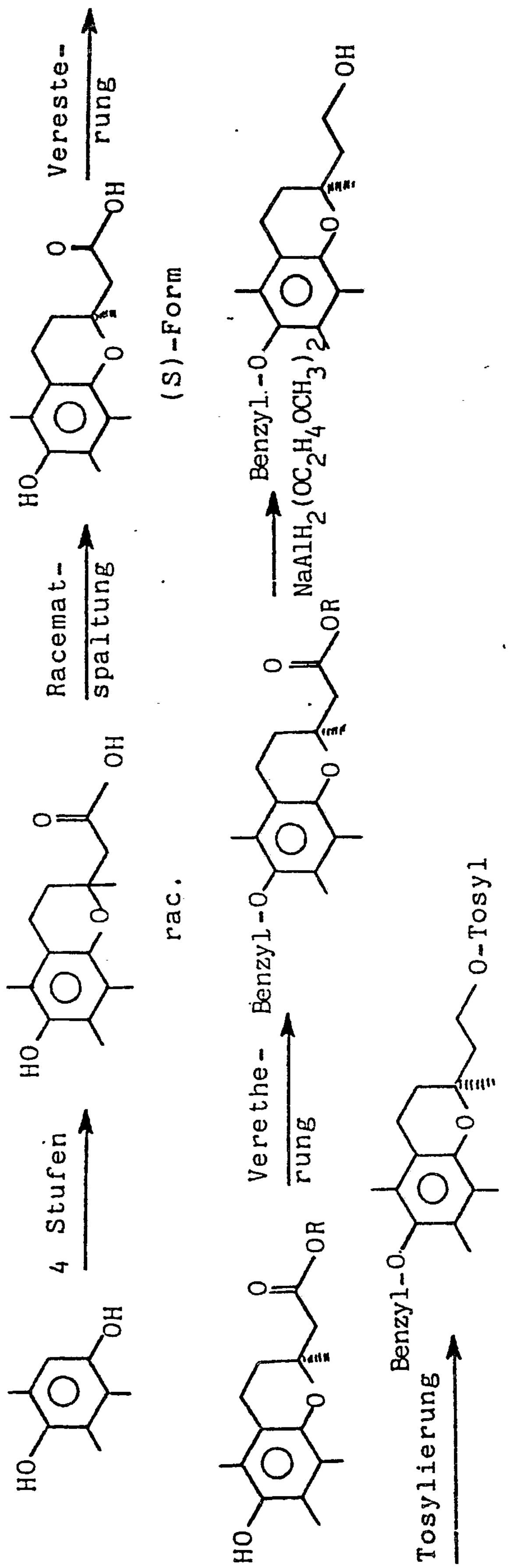


Da dieses Verfahren äusserst umständlich ist und überdies in der Hydrierstufe die Verwendung teurer Hydride, wie $NaAlH_2(OC_2H_4OCH_3)_2$, erfordert, war es die Aufgabe der Erfindung auf einfachere und billigere Weise zu (2R, 4′R, 8′R)- oder (2R, 4′RS, 8′RS)-α-Tocopherol kupplungsfähige Chromanderivate zugänglich zu machen.

Es wurde nun gefunden, dass man die eingangs definierten Chromanderivate Ia und Ib erhält, wenn man das Racemat I′

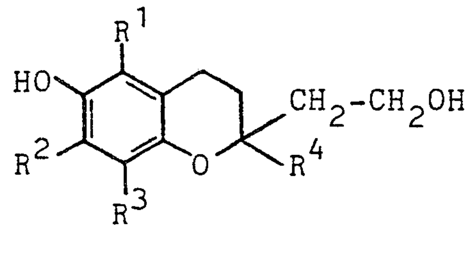

(I')

a) mit einer Carbonsäure der Formel II

R′-COOH (II),

worin R′ einen $C_1$- bis $C_4$-Alkylrest bedeutet, oder einem niederen Alkylester einer solchen Säure partiell in den Ester I″

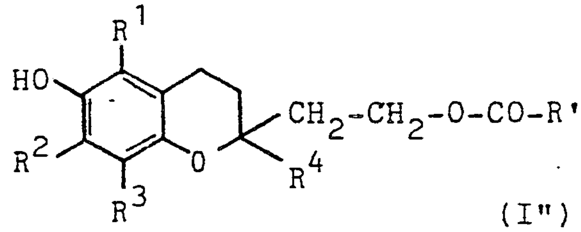

(I")

überführt, diesen mit einem optisch aktiven Carbonsäurehalogenid der allgemeinen Formel III

```
R⁵-CH-CO-X          (III),
   |
   O-Ar
```

in der $R^5$ eine Methylgruppe, X Cl oder Br und Ar eine Phenyl-, α-Naphthyl- oder β-Naphthylgruppe bedeutet, die ihrerseits durch eine Methylgruppe, Chlor oder Brom substituiert sein kann oder mit dem entsprechenden Carbonsäureanhydrid der allgemeinen Formel V

```
R⁵-CH-CO-O-CO-CH-R⁵          (V),
   |           |
   O-Ar        O-Ar
```

zu den Chromanderivaten IV

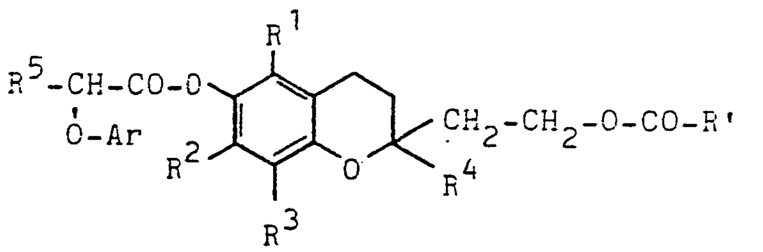
(IV)

acyliert, das aus zwei diastereomeren Estern bestehende IV durch fraktionierte Kristallisation spaltet, die reinen Diastereomeren wie üblich zu den Alkoholen der Formeln Ia bzw. Ib hydrolysiert, und diese gegebenenfalls in an sich bekannter Weise in die übrigen Verbindungen der Formeln Ia und Ib überführt oder wenn man

b) das Racemat I′ mit einer optisch aktiven Carbonsäure III′ oder einem niederen Alkylester hiervon

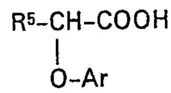
(III′)

in den Ester I‴

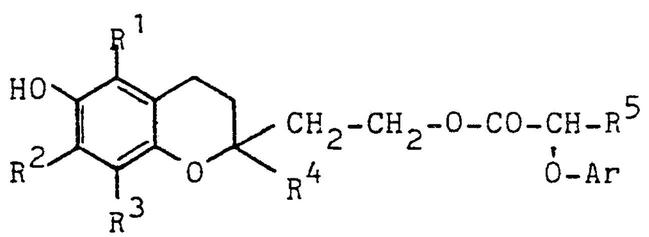
(I‴)

überführt, diesen gegebenenfalls mit einem Carbonsäurehalogenid der Formel R′-CO-X, worin X Cl, Br oder J bedeutet, oder dem entsprechenden Carbonsäureanhydrid zu IV′ acyliert

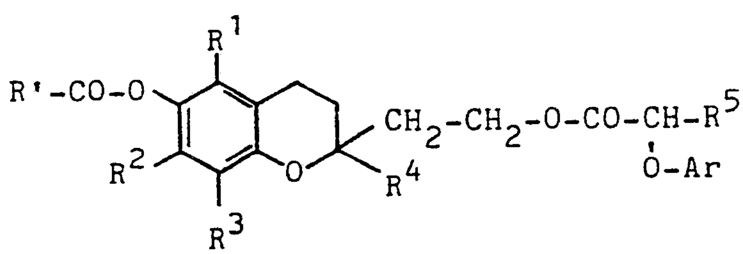
(IV′)

und das aus zwei Diastereomeren bestehende I‴ bzw. IV′ durch fraktionierte Kristallisation spaltet, die reinen Diastereomeren wie üblich zu den Alkoholen der Formeln Ia bzw. Ib hydrolysiert und diese gegebenenfalls in an sich bekannter Weise in die übrigen Verbindungen der Formeln Ia und Ib überführt.

In beiden Varianten des erfindungsgemässen Verfahrens geht man von dem racemischen Chromanderivat I′ aus, welches aus der DE-OS 3 010 505 bekannt ist und durch Friedel-Craffts-Addition des 1-Vinyl-propan-1,3-diols

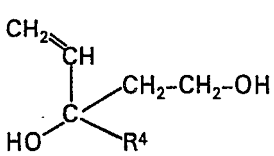

an das Hydrochinon

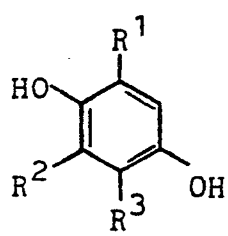

leicht zugänglich ist.

I′ hat zwei funktionelle Hydroxylgruppen, nämlich eine alkoholische und eine phenolische, die sich wegen ihrer unterschiedlichen Reaktivität leicht nach allgemeinen Gesetzmässigkeiten partiell verestern lassen.

Die Veresterung der alkoholischen Hydroxylgruppe nimmt man mit der freien Säure oder

einem niederen Alkylester als milden Veresterungsagentien vor, wogegen es zur daran anschliessenden Veresterung der phenolischen Hydroxylgruppe der entsprechenden Säurehalogenide als stärkeren Veresterungsagentien bedarf.

Nach dem erfindungsgemässen Verfahren kann der Säurerest der definitionsgemässen optisch aktiven Säure III' sowohl mit der alkoholischen als auch der phenolischen Hydroxylgruppe von I' verknüpft werden, wie es den beiden Verfahrensvarianten a) und b) entspricht. In beiden Fällen erhält man diastereomere Bisester, nämlich IV und IV', die sich wider Erwarten leicht durch fraktionierte Kristallisation in ihre Diastereomeren spalten lassen.

Beiden Verfahrensvarianten gemeinsam ist die Einführung des Restes der optisch aktiven Säure III', wogegen die Veresterung mit der optisch inaktiven Säure II nur bei Variante a) erforderlich ist und bei Variante b) entfallen kann.

Als optisch inaktive niedere Carbonsäure II kommt insbesondere die Essigsäure in Betracht, daneben aber auch die Propionsäure, die Buttersäure und die Isobuttersäure.

Als Säuren III' – sie entsprechen den Säureresten von III – eignen sich besonders solche, in denen $R^5$ für eine Methylgruppe steht. Die Arylgruppe Ar ist im einfachsten Fall eine Phenylgruppe, aber auch die α- und β-Naphthylgruppen, sowie substituierte Phenyl- und Naphthylgruppen kommen in Betracht. Als Substituenten an Ar seien genannt: $C_1$- bis $C_4$-Alkylgruppen, $C_1$- bis $C_4$-Alkoxygruppen, Chlor, Brom sowie die Cyan und die Nitrogruppe. Vorzugsweise sollen die Phenyl- bzw. die Naphthylgruppen nicht mehr als zwei dieser Substituenten tragen.

Die optisch aktiven Säuren III' sind bekannt oder nach bekannten Methoden zugänglich. Sie sind unseres Wissens noch nicht für Racematspaltungen eingesetzt worden. Die Säurederivate III sind aus den freien Säuren wie üblich erhältlich, z.B. durch Umsetzung mit beispielsweise Thionylchlorid. Weiterhin geeignete optisch aktive Reagentien sind die entsprechenden Anhydride der Formel V.

Besonders bevorzugt als Säuren III' bzw. als Säurehalogenide III sind die optisch aktiven Formen der
1-Phenoxy-propionsäure
2-(2'-Methyl-4'-chlor-phenoxy)-propionsäure
und der 1-(1-Naphthoxy)-propionsäure.

Die beiden Verfahrensvarianten seien im einzelnen wie folgt erläutert:

Nach der Ausführungsform a) nimmt man die Veresterung von I' mit der $C_2$- bis $C_4$-Fettsäure II wie üblich vorzugsweise in organischer Lösung vor. Als Lösungsmittel eignen sich beispielsweise z.B. Benzol, Toluol, Essigsäureester oder Gemische dieser Lösungsmittel.

Als Veresterungskatalysatoren können beispielsweise Schwefelsäure oder p-Toluolsulfonsäure verwendet werden.

Die gleichen Bedingungen gelten auch für den Fall, dass man anstelle der freien Säure deren niedere Alkylester, vorzugsweise deren Methylester einsetzt. Die Umesterung bietet den Vorteil, dass keine wässrige Phase entsteht, wenn die Reaktion in einem wasserunlöslichen Lösungsmittel ausgeführt wird.

Man kann die entstehenden Ester I'' isolieren, jedoch ist es auch möglich, die Acylierung mit III unmittelbar anzuschliessen, sofern keine nennenswerten Mengen von Wasser oder Alkoholen mehr zugegen sind.

Die Acylierung der phenolischen Hydroxylgruppe mit dem Säurechlorid III nimmt man vorzugsweise – wie üblich – in Gegenwart von äquimolaren Mengen einer tertiären Stickstoffbase, wie Pyridin, als Säurefänger vor. Nach beendeter Acylierung versetzt man das Reaktionsgemisch wie üblich mit Wasser, trennt die organische Phase wie üblich ab und entfernt hiervon gegebenenfalls das Lösungsmittel.

Der aus den Diastereomeren IV bestehende Rückstand wird in einem Lösungsmittel aufgenommen und wie üblich fraktioniert kristallisiert.

Als Lösungsmittel eignen sich die im Zusammenhang mit der Veresterungsreaktion genannten Lösungsmittel sowie $C_1$- bis $C_4$-Alkanole, insbesondere Methanol und Ethanol.

Entsprechend üblicher Arbeitstechnik löst man nach Einengen der Extrakte auskristallisiertes IV zunächst vollständig in dem heissen Lösungsmittel, aus dem man dann beim Abkühlen das eine Diastereomere durch Kristallisation gewinnt. Gegebenenfalls ist die Kristallisation ein oder zwei mal zu wiederholen, wie es dem gewünschten Reinheitsgrad entspricht.

Die nunmehr getrennten Diastereomeren werden schliesslich wie üblich verseift, und zwar vorzugsweise mit wässrig-alkoholischer Kalilauge. Danach extrahiert man die Alkohole der Formeln Ia bzw. Ib wie üblich mit einem Lösungsmittel, z.B. Methylenchlorid, welches mit der wässrig-alkoholischen Phase nicht mischbar ist.

Man erhält die Alkohole der Formeln Ia bzw. Ib nach Methode a), also über Veresterung, Umkristallisation und Verseifung in der Regel in Ausbeuten von 25 bis 30%, bezogen auf das racemische I', d.h. 50 bis 60% der theoretisch möglichen Menge.

Methode b) entspricht präparativ der Methode a) mit dem Unterschied, dass hier die alkoholische Hydroxylgruppe von I' zunächst mit der freien optisch aktiven Säure III' oder einem niederen Alkylester hiervon verestert wird. Die Acylierung der phenolischen Hydroxylgruppe mit dem Säurehalogenid II' erübrigt sich prinzipiell, ist andererseits aber von Vorteil, da die Bisester häufig besser kristallisieren als die Monoester.

Ausserdem ergibt sich hierbei durch die besonderen Löslichkeitsverhältnisse von Monoestern und Bisestern unerwartet ein entscheidender Vorteil des neuen Racematspaltungsverfahrens. Verestert man nämlich das Racemat I' zunächst beispielsweise mit (−)-1-(1-Naphthoxy)-propionsäure, arbeitet das Reaktionsgemisch wie üblich durch Ausschütteln mit Wasser und Extrahieren auf, engt die vereinigten Extrakte ein, löst den erhaltenen Rohester in Methanol oder Ethanol auf und impft an, so kristallisiert langsam (R)-2,5,7,8-Tetramethyl-6-hydroxy-2-chromanyl-ethyl-(−)-1-(1)-naphthoxy-propionat aus, aus dem durch Verseifung das (R)-2,5,7,8-Tetramethyl-6-hydroxy-2-(2-hydroxy-ethyl)-chroman gewonnen werden kann (vgl. Beispiel 5). Wird nun die abgetrennte Mutterlauge eingeengt und das nunmehr an dem (S)-2,5,7,8-Tetramethyl-6-hydroxy-2-(2-hydroxy-ethyl)-chromanester angereicherte Diasteromerengemisch beispielsweise mit Acetylchlorid oder Acetanhydrid in 6-Stellung acetyliert, das Reaktionsgemisch wie üblich aufgearbeitet und der erhaltene rohe Bisester in heissem Methanol (oder Ethanol) gelöst, so kristallisiert überraschenderweise zunächst der Bisester des (S)-Chromanderivats aus, aus dem durch Verseifung das (S)-2,5,7,8-Tetramethyl-6-hydroxy-2-(-2-hydroxy-ethyl)-chroman hergestellt werden kann.

Alle folgenden Verfahrensschritte der Racematspaltung verlaufen analog Ausführungsform a). Eine besonders vorteilhafte Variante des erfindungsgemässen Verfahrens besteht dementsprechend darin, dass man das Racemat I′ mit einer Carbonsäure III′ oder einem niederen Alkylester hiervon in den Ester I‴ überführt, aus dem erhaltenen Gemisch der diastereomeren Ester I‴, die beim Lösen in Alkoholen zunächst auskristallisierende (R)-Form abtrennt, aus den abgetrennten Mutterlaugen den verbleibenden, überwiegend die (S)-Form enthaltenden Monoester mit einem Carbonsäurehalogenid der Formel R′–CO–X, worin X Cl, Br oder J bedeutet, oder dem entsprechenden Carbonsäureanhydrid in 6-Stellung verestert und aus der überwiegend die (S)-Form des Chromanderivats IV′ enthaltenden Lösung der Diastereomeren IV′ die nunmehr bevorzugt auskristallisierende (S)-Form von IV′ isoliert, die reinen Diastereomeren wie üblich zu den Alkoholen der Formeln Ia bzw. Ib hydrolysiert und diese gegebenenfalls in an sich bekannter Weise in die übrigen Verbindungen der Formeln Ia und Ib überführt.

Abschliessend lässt sich über das neue Verfahren zur Spaltung von racemischem 2,5,7,8-Tetramethyl-6-hydroxy-2-(2-hydroxy-ethyl)-chroman in seine (R)- und (S)-Form folgendes sagen: Die optisch aktiven Säuren III′ bzw. ihre Derivate sind noch nicht zur Racematspaltung verwendet worden. Es war überraschend, dass die erfindungsgemässe Racematspaltung mittels dieser optisch aktiven Säuren bzw. deren Derivaten so vorteilhaft gelingt, da Versuche, das Chromanylethylacetat mit Hilfe von aus der Literatur für die Racematspaltung von phenolischen Verbindungen bekannter optisch aktiver Säurederivate, wie dem (−)-Menthyl-oxyacetylchlorid (vgl. S. Fujise et al., Chem. Ber. 69 (1936), Seite 1893 und A.E. Knauf et al., Am. Chem. Soc. 56, (1934), Seite 2109) zu spalten, nicht befriedigend verliefen. Es war weiterhin überraschend, dass die Racematspaltung so vorteilhaft verläuft, auch wenn der Rest der optisch aktiven Säure mit der phenolischen Hydroxylgruppe des Chromanrings verknüpft und damit sehr weit von dem chiralen C-Atom des Chromanderivats entfernt ist. Es war darüber hinaus nicht vorhersehbar, dass es – bedingt durch die besonderen Löslichkeitsverhältnisse der Chromanylethanolmonoester bzw. -diester – möglich sein würde, mit Hilfe von nur einer optisch aktiven Form der Säure auf einfache Weise beide diastereomeren Formen des Chromanderivats zu isolieren (vgl. Beispiel 3 und Beispiel 5), was natürlich auch bedeutet, dass man das begehrtere (S)-2,5,7,8-Tetramethyl-6-hydroxy-2-(2-hydroxy-ethyl)-chroman z.B. sowohl mittels der (−)1-(1-Naphthoxy)-propionsäure (vgl. Beispiel 3) als auch mittels der (+)-1-(1-Naphthoxy)-propionsäure (vgl. Beispiel 6) erhalten kann.

Die Überführung der bei der Racematspaltung zunächst erhaltenen Chromanderivate der Formeln Ia und Ib, in denen X für OH steht, in die übrigen definitionsgemässen Chromanderivate erfolgt in an sich bekannter Weise, so dass sich detaillierte Angaben hierüber erübrigen.

Beispielsweise erhält man (S)- bzw. (R)-2,5,7,8-Tetramethyl-6-hydroxy-2-(2-brom-ethyl)-chroman durch Umsetzen des entsprechenden -2-(2-hydroxy-ethyl)-chromans mit einer Lösung von Triphenylphosphin und Brom in wasserfreiem Methylenchlorid und (S)- bzw. (R)-2,5,7,8-Tetramethyl-6-hydroxy-2-(2-chlor-ethyl)-chroman durch Umsetzen des entsprechenden -2-(2-hydroxy-ethyl)-chromans mit $CCl_4$ und Triphenylphosphin. Bezüglich näherer Einzelheiten dieser Verfahren verweisen wir auf E. Schacht in «Kontakte», Heft 3, Seite 9, 1974, sowie hierin zitierte Literatur.

(S)- bzw. (R)-2,5,7,8-Tetramethyl-6-hydroxy-2-(2-tert.-butoxy-ethyl)-chroman erhält man durch Umsetzen einer Lösung des entsprechenden -2-(2-hydroxy-ethyl)-chromans in Methylenchlorid mit Isobuten in Gegenwart von geringen Mengen Schwefelsäure analog dem Verfahren von H. C. Beyerman et al., in Rec. Trav. Chim. 84 (1965) Seite 203.

Die vorliegende Erfindung ermöglicht die Herstellung von zu α-Tocopherol kupplungsfähigen optisch aktiven Chromanderivaten in einer im Vergleich mit dem Stand der Technik sehr kurzen und leicht durchführbaren Synthesestufenfolge:

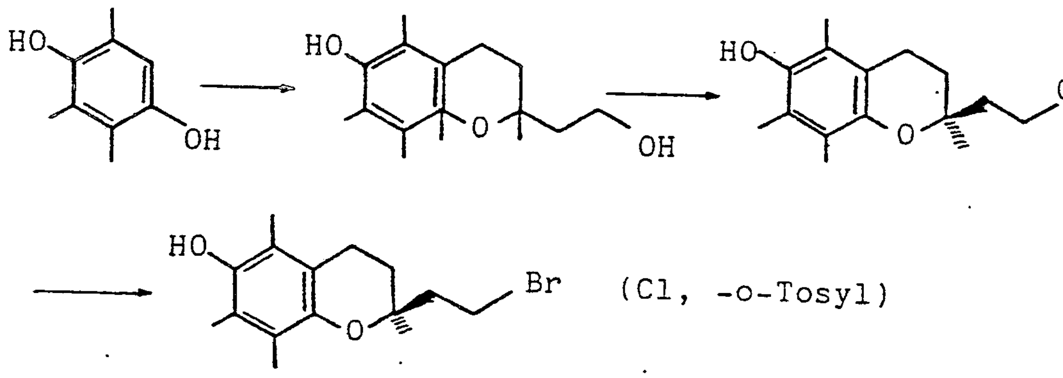


Die erfindungsgemässen Chromanderivate der Formel Ia, in der X für Cl, Br oder O-Tosyl stehen, lassen sich gemäss dem Verfahren einer mit gleichem Datum eingereichten Patentanmeldung (vgl. EP-A 121 773) sehr vorteilhaft direkt mit einer entsprechenden $C_{14}$-Grignardverbindung unter Katalyse mit einem Di-(alkalimetall)-tetrahalogen-kuprat nach Schlosser-Fouquet zu einem α-Tocopherolisomeren verknüpfen.

Beispiel 1

Herstellung von (S)-2,5,7,8-Tetramethyl-6-hydroxy-2-(2-hydroxy-ethyl)-chroman nach Variante a)

a) 200 g (800 mmol) des racemischen 2,5,7,8-Tetramethyl-6-hydroxy-2-(2-hydroxyethyl)-chromans (I′) wurden wie üblich mit 100 g Eisessig in Gegenwart von 4 g p-Toluolsulfonsäure und 1,5 l Essigsäureethylester im Laufe von 6 h unter Rückflussbedingungen verestert.

Die übliche Aufarbeitung lieferte den Essigsäureester von I′ (2,5,7,8-Tetramethyl-6-hydroxy-2-(2-acetoxyethyl)-chroman in Form eines gelben Öls, das aus Ethanol umkristallisiert wurde. Fp. = 74°C, Ausbeute = 85% der Theorie.

b) Eine Lösung von 100 g des gemäss Beispiel 1a) hergestellten Esters in 250 ml Pyridin wurde bei 0°C allmählich mit L-(4-Chlor-2-methyl)-phenoxy-propionylchlorid, das durch 3stündiges Erhitzen von 80 g (374 mmol) L-(4-Chlor-2-methyl)-phenoxypropionsäure mit 125 g Thionylchlorid auf 50°C und Abdestillieren des überschüssigen Thionylchlorids erhalten worden war, versetzt.

Anschliessend wurde das Reaktionsgemisch noch 16 h bei Raumtemperatur (RT) gerührt und dann in Eiswasser gegeben. Das erhaltene wässrig-organische Gemisch wurde mit Methylenchlorid extrahiert, die erhaltene organische Phase mit 5 n HCl, Wasser, verd. Sodalösung und erneut mit Wasser gewaschen; getrocknet und eingeengt. Das beim Einengen verbliebene Öl wurde in 500 ml Methanol aufgenommen, die Lösung zum Sieden erhitzt und dann langsam abkühlen lassen. Der hierbei erhaltene Kristallbrei wurde abfiltriert und unter Erhitzen bis zur Lösung mit Methanol versetzt. Der beim Abkühlen der methanolischen Lösung auskristallisierte Niederschlag wurde abfiltriert, ein weiteres Mal aus Methanol umkristallisiert und getrocknet. Man erhielt 58 g des Gemischs der diastereomeren Bisester.

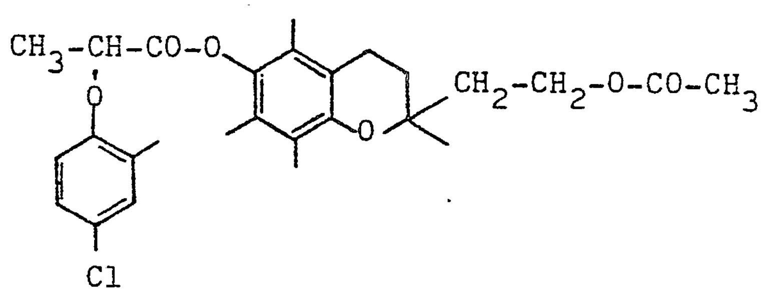

vom Schmelzpunkt F = 122°C und $[\alpha]_D^{20} = -63{,}8°$ (c = 2, Aceton). Die Ausbeute betrug somit 35% der Theorie, bezogen auf den in dieser Stufe eingesetzten Chromanylester, die optische Reinheit betrug 100%.

58 g (119 mmol) des gemäss Beispiel 1b) erhaltenen reinen Estergemisches wurden mit 500 ml Methanol und 132 ml in Kalilauge 2 h zum Sieden erhitzt. Die übliche Aufarbeitung dieses Verseifungsgemisches durch Extraktion mit Methylenchlorid lieferte das gewünschte S-2,5,7,8-Tetramethyl-6-hydroxy-2-(2-hydroxyethyl)-chroman in einer Gesamtausbeute von 27% der Theorie, bezogen auf das eingesetzte racemische Hydroxyethylchroman. Fp. = 155°C; $[\alpha]_D^{20} = -6{,}95°$ (c = 2; Ethanol).

Beispiel 2

Herstellung von R-2,5,7,8-Tetramethyl-6-hydroxy-2-(2-hydroxy-ethyl)-chroman

Diese Verbindung wurde analog Beispiel 1 mit D-(4-Chlor-2-methyl-phenoxy)-propinylchlorid als Spaltungsreagenz hergestellt. Ausbeute 29% der Theorie, bezogen auf das eingesetzte racemische Hydroxyethylchroman. Fp. = 155°C; $[\alpha]_D^{20}$ +7,00° (c = 2; Aceton).

Beispiel 3

Herstellung von S-2,5,7,8-Tetramethyl-6-hydroxy-2-(2-hydroxy-ethyl)-chroman nach Variante b) mit Veresterung der phenolischen Hydroxylgruppe

10 g (40 mmol) racemisches 2,5,7,8-Tetramethyl-6-hydroxy-2-(2-hydroxy-ethyl)-chroman wurden in Gegenwart von 200 ml Toluol und 0,1 g p-Toluolsulfonsäure durch 4stündiges Erhitzen unter Auskreisen von Wasser mit 8,6 g (40 mmol) (−)-1-Naphthoxy-propionsäure verestert. Das erhaltene Reaktionsgemisch wurde nach dem Abkühlen mit Natriumbicarbonatlösung und Wasser ausgeschüttelt, und die organische Phase wurde eingeengt. Der so erhaltene rohe Ester wurde sodann in 40 ml Pyridin gelöst, die Lösung mit 4 g Acetanhydrid versetzt und 24 h bei RT stehenlassen. Das nach üblicher Aufarbeitung gewonnene rohe Bisestergemisch (158 g) wurde 2mal der fraktionierten Kristallisation aus Ethanol unterworfen. Die Ausbeute an der S-Form betrug 29% der Theorie, bezogen auf das Racemat, d.h. 58% der theoretisch möglichen Menge; Fp. = 145°C; $[\alpha]_D^{20}$ = −39,0° (c = 2; Chloroform).

Die alkalische Verseifung des Bisesters analog Beispiel 1c lieferte das gewünschte S-2,5,7,8-Tetramethyl-6-hydroxy-2-(2-hydroxy-ethyl)-chroman in 26%iger Ausbeute, bezogen auf die eingesetzte racemische Verbindung. $[\alpha]_D^{20}$ = −7,0° (c = 2; Ethanol). Fp. = 155°C.

Beispiel 4

Herstellung von R-2,5,7,8-Tetramethyl-6-hydroxy-2-(2-hydroxy-ethyl)-chroman

Diese Verbindung wurde analog Beispiel 3 mit (+)-1-Naphthoxy-propionsäure als Spaltreagenz hergestellt.

Beispiel 5

Herstellung von R-2,5,7,8-Tetramethyl-6-hydroxy-2-(2-hydroxy-ethyl)-chroman nach Variante b) ohne Veresterung der phenolischen Hydroxylgruppe

10 g (40 mmol) racemisches 2,5,7,8-Tetramethyl-6-hydroxy-2-(2-hydroxy-ethyl)-chroman wurden durch 4stündiges Erhitzen unter Wasserauskreisen in Gegenwart von 200 ml Toluol und 0,1 g p-Toluolsulfonsäure mit 8,6 g (40 mmol) (−)-1-Naphthoxy-propionsäure verestert. Der durch übliche Aufarbeitung erhaltene Rohester (16 g) wurde sodann in 90 ml Methanol gelöst.

Diese Lösung wurde mit 20 mg (R)-2,5,7,8-Tetramethyl-6-hydroxy-2-chromanyl-ethyl-(−)-1-naphthoxypropionat angeimpft, wonach sich im Laufe von 2 Tagen bei −20°C ein Niederschlag bildete. Dieser wurde abgetrennt und auf gleiche Weise nochmals umkristallisiert.

Ausbeute 26%, bezogen auf das eingesetzte racemische Chroman. Fp. = 121°C; $[\alpha]_D^{25}$ = −41° (c = 2; $CHCl_3$).

4 g des so dargestellten Esters wurden in 140 ml Methanol gelöst und mit 9,0 ml 1 m KOH 1,5 h unter Rückfluss zum Sieden erhitzt. Nach Aufarbeitung analog Beispiel 1c erhielt man 2,1 g R-2,5,7,8-Tetramethyl-6-hydroxy-2-(2-hydroxy-ethyl)-chroman. Fp. = 155°C; $[\alpha]_D^{20}$ = +6,95° (c = 2; Ethanol).

Beispiel 6

Herstellung von S-2,5,7,8-Tetramethyl-6-hydroxy-2-(2-hydroxy-ethyl)-chroman

Diese Verbindung wurde analog Beispiel 5 mit (+)-1-Naphthoxy-propionsäure als Spaltreagenz hergestellt.

Beispiel 7

Herstellung von S-2,5,7,8-Tetramethyl-6-hydroxy-2-(2-hydroxy-ethyl)-chroman

Die vereinten Mutterlaugen aus Beispiel 5 wurden eingeengt, mit 3 g Acetanhydrid und 30 ml Pyridin versetzt und 24 h stehenlassen. Aufarbeitung des Reaktionsansatzes durch Hydrolyse mit Eiswasser, Extraktion mit Methylenchlorid, Waschen und Einengen der organischen Phase und 2maliges Umkristallisieren des gebildeten Niederschlages aus Ethanol ergab 6,6 g S-2,5,7,8-Tetramethyl-6-acetoxy-2-chromanyl-ethyl-(−)-1-naphthoxypropionat vom Schmelzpunkt Fp. = 145°C und $[\alpha]_D^{20}$ = −38,7° (c = 2; Chloroform). Die Ausbeute betrug 34%, bezogen auf in Beispiel 5 eingesetztes Chromanol.

4 g des so hergestellten Esters wurden in 140 ml Methanol gelöst und mit 16,4 ml 1 m KOH 1,5 h unter Rückfluss zum Sieden erhitzt. Nach Aufarbeitung analog Beispiel 1c erhielt man das gewünschte (S)-Chromanylethanol in einer Ausbeute von 31% der Theorie, bezogen auf eingesetztes racemisches Chromanol.

Beispiel 8

5,5 g Triphenylphosphin wurden in 100 ml wasserfreiem Methylenchlorid gelöst. Dazu wurden 3,4 g Brom getropft, das Reaktionsgemisch 30 min bei RT gerührt, mit 5 g (S)-2,5,7,8-Tetramethyl-6-hydroxy-2-(2-hydroxy-ethyl)-chroman versetzt und 1 h unter Rückfluss zum Sieden erhitzt. Anschliessend wurde auf Sodalösung gegossen und die wässrige Phase mit Methylenchlorid extrahiert. Die vereinten organischen Phasen wurden mit Kochsalzlösung gewaschen, getrocknet, eingeengt und der Rückstand aus Methanol umkristallisiert. Man erhielt 5,3 g (S)-2,5,7,8-Tetramethyl-6-hydroxy-2-(2-brom-ethyl)-chroman. Fp. = 120°C; $[\alpha]_D^{20}$ = −15° (c = 2; MeOH).

Beispiel 9

Aus (R)-2,5,7,8-Tetramethyl-6-hydroxy-2-(2-hydroxy-ethyl)-chroman wurde analog Beispiel 8 das R-2,5,7,8-Tetramethyl-6-hydroxy-2-(2-brom-ethyl)-chroman hergestellt. Fp. = 120°C; $[\alpha]_D^{20}$ = +15,1° (c = 2; MeOH).

Beispiel 10

Eine Mischung aus 2 g (S)-2,5,7,8-Tetramethyl-6-hydroxy-2-(2-hydroxy-ethyl)-chroman, 8 g Tetrachlorkohlenstoff und 2,6 g Triphenylphosphin wurden 3 h unter Rückfluss zum Sieden erhitzt.

Anschliessend liess man abkühlen, saugte den gebildeten Niederschlag ab und chromatographierte das Filtrat an Kieselgel im Lösungsmittelgemisch Hexan/Aceton (5:1). Man erhielt 1,7 g (S)-2,5,7,8-Tetramethyl-6-hydroxy-2-(2-chlorethyl)-chroman. Fp. = 111°C; $[\alpha]_D^{20} = -9,1°$ (c = 2; Chloroform).

Beispiel 11

Aus (R)-2,5,7,8-Tetramethyl-6-hydroxy-2-(2-hydroxy-ethyl)-chroman wurde analog Beispiel 10 das (R)-2,5,7,8-Tetramethyl-6-hydroxy-2-(2-chlor-ethyl)-chroman hergestellt. Fp. = 111°C; $[\alpha]_D^{20} = +9,0°$ (c = 2; Chloroform).

Beispiel 12

5 g (S)-2,5,7,8-Tetramethyl-6-hydroxy-2-(2-hydroxy-ethyl)-chroman wurden in 250 ml Methylenchlorid suspendiert und durch die Suspension Isobuten durchgeleitet. Nach 15 min wurde 1 ml konz. Schwefelsäure hinzugefügt. Dann wurde weitere 4 h bei Raumtemperatur unter Durchleiten eines schwachen Isobutenstroms gerührt. Anschliessend liess man den Ansatz verschlossen über Nacht stehen. Die organische Phase wurde mit Natriumhydrogencarbonatlösung und mit Wasser gewaschen, getrocknet und eingeengt. Das Rohprodukt wurde an Kieselgel mit dem Lösungsmittelgemisch Hexan/Aceton (5:1) gereinigt. Man erhielt 4,5 g (S)-2,5,7,8-Tetramethyl-6-hydroxy-2-(2-tert.-butoxy-ethyl)-chroman als farbloses Öl. $[\alpha]_{365}^{20} = +4,23°$ (c = 2; Ethanol).

Beispiel 13

Aus (R)-2,5,7,8-Tetramethyl-6-hydroxy-2-(2-hydroxy-ethyl)-chroman wurde analog Beispiel 12 das (R)-2,5,7,8-Tetramethyl-6-hydroxy-2-(2-tert.-butoxy-ethyl)-chroman erhalten. $[\alpha]_D^{20} = 4,2°$ (c = 2; Ethanol).

Beispiel 14

Analog Beispiel 1a) wurde aus (S)-2,5,7,8-Tetramethyl-6-hydroxy-2-(2-hydroxy-ethyl)-chroman das (S)-2,5,7,8-Tetramethyl-6-hydroxy-2-(2-acetoxy-ethyl)-chroman erhalten. Fp. = 80,5°C; $[\alpha]_D^{20} = -0,1°$ (c = 2; Chloroform).

**Patentansprüche**

1. Verfahren zur Herstellung von neuen optisch aktiven Chromanderivaten der allgemeinen Formeln Ia und Ib

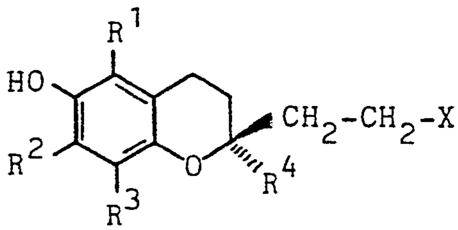

(Ia)

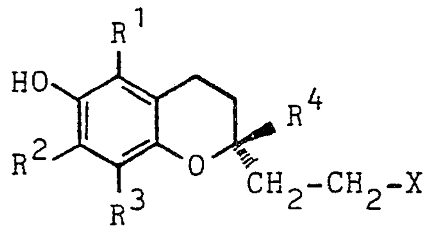

(Ib)

in denen die Reste $R^1$ bis $R^4$ Wasserstoff oder eine Methylgruppe bedeuten und X für -OH, -O-CO-$R^6$-, O-Tosyl, O-Mesyl, O-Benzolsulfonyl, Cl, Br oder J stehen, worin $R^6$ eine $C_1$- bis $C_4$-Alkylgruppe bedeutet, dadurch gekennzeichnet, dass man das Racemat I′

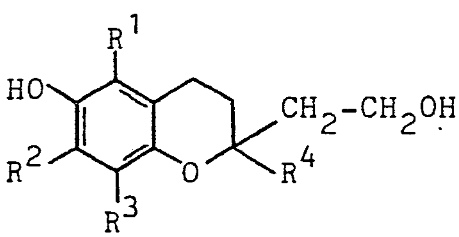

(I′)

a) mit einer Carbonsäure der Formel II

R′-COOH (II),

worin R′ einen $C_1$- bis $C_4$-Alkylrest bedeutet, oder einem niederen Alkylester einer solchen Säure partiell in den Ester I″

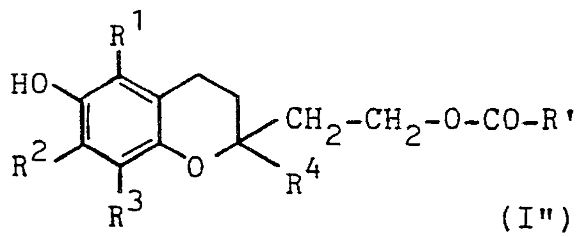

(I″)

überführt, diesen mit einem optisch aktiven Carbonsäurehalogenid der allgemeinen Formel III

$R^5$-CH-CO-X (III),
|
O-Ar

in der $R^5$ eine Methylgruppe X, Cl oder Br oder Ar eine Phenyl-, α-Naphthyl- oder β-Naphthylgruppe bedeutet, die ihrerseits durch eine $C_1$- bis $C_4$-Alkylgruppe, Chlor oder Brom substituiert sein kann, oder mit dem entsprechenden Carbonsäureanhydrid der allgemeinen Formel V

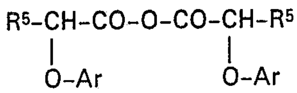 (V),

zu den Chromanderivaten IV

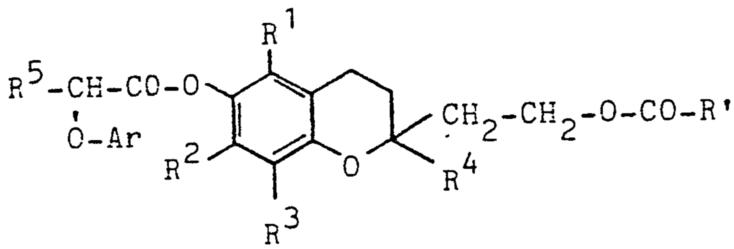 (IV)

acyliert, das aus zwei diastereomeren Estern bestehende IV durch fraktionierte Kristallisation spaltet, die reinen Diastereomeren wie üblich zu den Alkoholen der Formeln Ia bzw. Ib hydrolysiert und diese gegebenenfalls in an sich bekannter Weise in die übrigen Verbindungen der Formeln Ia und Ib überführt, oder dass man

b) das Racemat I′ mit einer Carbonsäure III′ oder einem niederen Alkylester hiervon

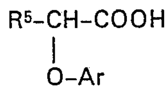 (III′)

in den Ester I‴

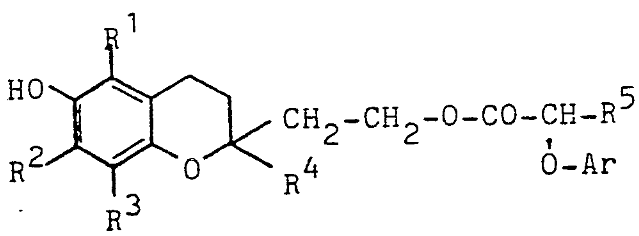 (I‴)

überführt, diesen gegebenenfalls mit einem Carbonsäurehalogenid der Formel R′-CO-X, worin X Cl, Br oder J bedeutet, oder dem entsprechenden Carbonsäureanhydrid zu IV′ acyliert

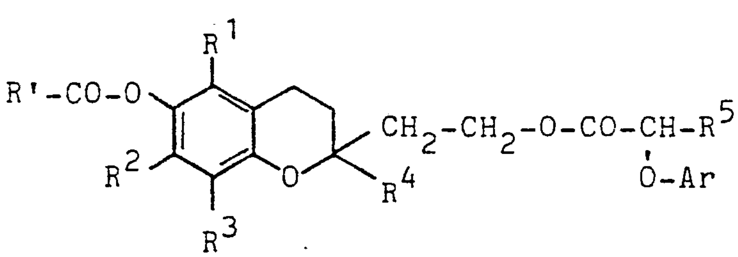 (IV′)

und das aus zwei Diastereomeren bestehende I‴ bzw. IV′ durch fraktionierte Kristallisation spaltet, die reinen Diastereomeren wie üblich zu den Alkoholen der Formeln Ia bzw. Ib hydrolysiert und diese gegebenenfalls in an sich bekannter Weise in die übrigen Verbindungen der Formeln Ia und Ib überführt.

2. Optisch aktive Chromanderivate der allgemeinen Formeln Ia und Ib

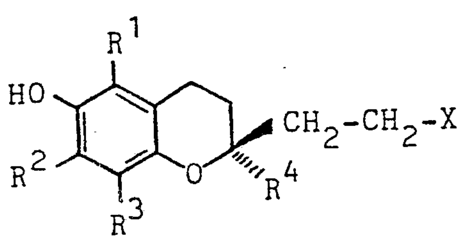

(Ia)

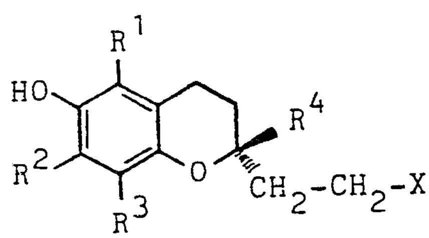

(Ib)

in denen $R^1$ bis $R^4$ für Methyl stehen und X -OH, Cl, Br, J, $-O-CH_3$, -O-tert.-butyl, -O-acetyl oder -O-p-Tosyl bedeutet.

3. (S)-2,5,7,8-Tetramethyl-6-hydroxy-2(2-hydroxy-ethyl)-chroman.

4. (R)-2,5,7,8-Tetramethyl-6-hydroxy)-2-(2-hydroxy-ethyl)-chroman.

5. (S)-2,5,7,8-Tetramethyl-6-hydroxy-2-(2-brom-ethyl)-chroman.

6. (R)-2,5,7,8-Tetramethyl-6-hydroxy-2-(2-brom-ethyl)-chroman.

7. (S)-2,5,7,8-Tetramethyl-6-hydroxy-2-(2-chlor-ethyl)-chroman.

8. (R)-2,5,7,8-Tetramethyl-6-hydroxy-2-(2-chlor-ethyl)-chroman.

9. (S)-2,5,7,8-Tetramethyl-6-hydroxy-2-(2-acetoxy-ethyl)-chroman.

10. (R)-2,5,7,8-Tetramethyl-6-hydroxy-2-(2-acetoxy-ethyl)-chroman.

11. Diastereomere Chromanylester der allgemeinen Formeln IV und IV′

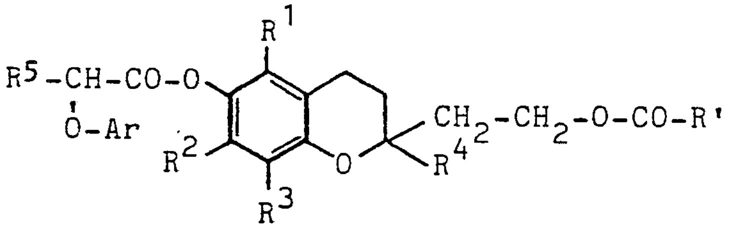

(IV)

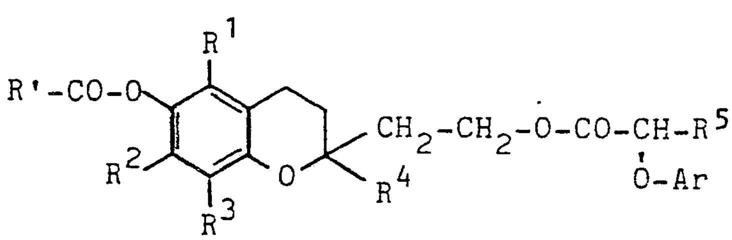

(IV')

in denen $R^5$ eine $C_1$- bis $C_3$-Alkylgruppe, R′ eine Methylgruppe und Ar eine Phenyl- oder Naphthylgruppe bedeutet, die ihrerseits durch eine Methylgruppe, Chlor oder Brom substituiert sein kann.

12. 2,5,7,8-Tetramethyl-6-[2′-(o-methyl-p-chlor-phenoxy)-propionyloxy]-2-(2-acetoxy-ethyl)-chroman.

13. 2,5,7,8-Tetramethyl-6-acetoxy-2-[2-(2′-α-naphthoxy)-propionyl-ethyl]-chroman.

14. 2,5,7,8-Tetramethyl-6-hydroxy-2-[2-(2′-α-naphthoxy)-propionyl-ethyl]-chroman.

**Claims**

1. A process for the preparation of novel optically active chroman derivatives of the general formulae Ia and Ib

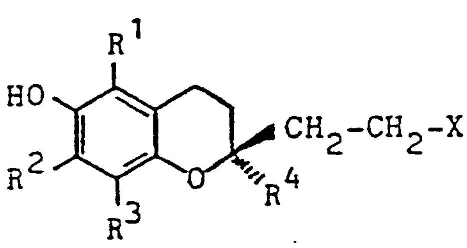

(Ia)

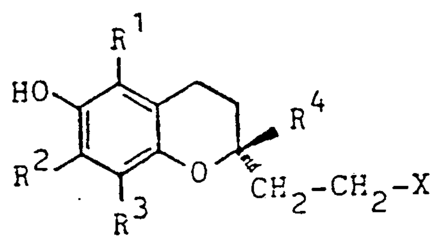

(Ib)

where $R^1$, $R^2$, $R^3$ and $R^4$ are each hydrogen or methyl and X is -OH, $-O-CO-R^6$, $-O-R^6$, -O-tosyl, -O-mesyl, -O-benzenesulfonyl, Cl, Br or I, where $R^6$ is $C_1$–$C_4$-alkyl, wherein

a) the racemate I′

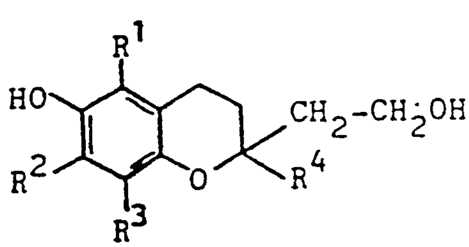

(I')

is selectively converted with a carboxylic acid of the formula II

R'-COOH (II),

whre R' is $C_1$–$C_4$-alkyl, or with a lower alkyl ester of such an acid, to the ester I''

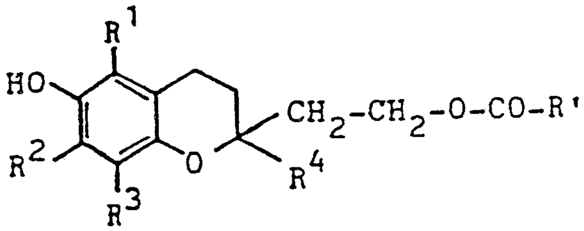

(I'')

this is acylated with an optically active carboxylic acid halide of the general formula III

$R^5$-CH-CO-X (III),
|
O-Ar

where $R^5$ is methyl, X is Cl or Br and Ar is phenyl, α-naphthyl or β-naphthyl, which in turn can be substituted by $C_1$–$C_4$-alkyl, chlorine or bromine, or with the corresponding carboxylic anhydride of the general formula V

$R^5$-CH-CO-O-CO-CH-$R^5$ (V),
| |
O-Ar O-Ar

to give the chroman derivative IV

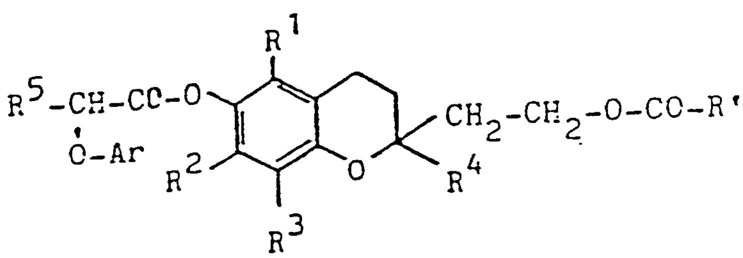

which consists of two diastereomeric esters, this is resolved by fractional crystallization, pure diastereomers are hydrolyzed in a conventional manner to give the alcohols of the formulae Ia and Ib, and, if desired, these are converted in a conventional manner to the other compounds of the formulae Ia and Ib, or

b) the racemate I' is converted with a carboxylic acid III'

$R^5$-CH-COOH (III')
|
O-Ar

or with a lower alkyl ester of this, to an ester I'''

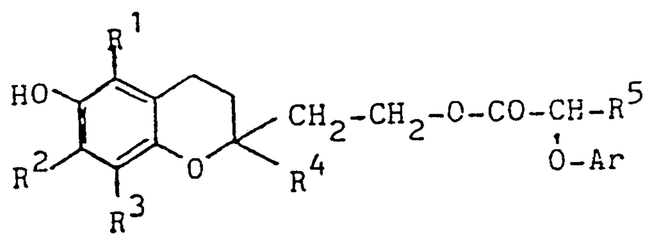

(I''')

this is, if desired, acylated with a carboxylic acid halide of the formula R'-CO-X, where X is Cl, Br or I, or with the corresponding carboxylic anhydride, to give IV'

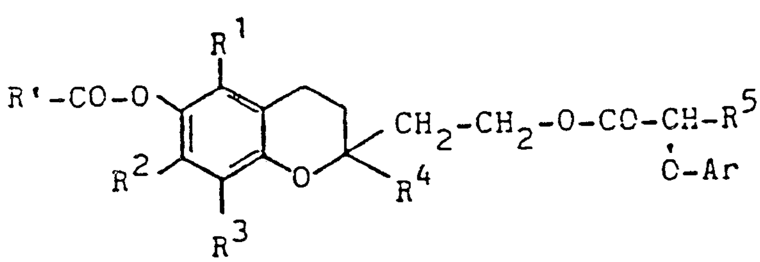

(IV')

I''' or IV', which consists of two diastereomers, is resolved by fractional crystallization, the pure diastereomers are hydrolyzed in a conventional manner to give the alcohols of the formulae Ia and Ib, and, if desired, these are converted in a conventional manner to the other compounds of the formulae Ia and Ib.

2. Optically active chroman derivatives of the general formulae Ia and Ib

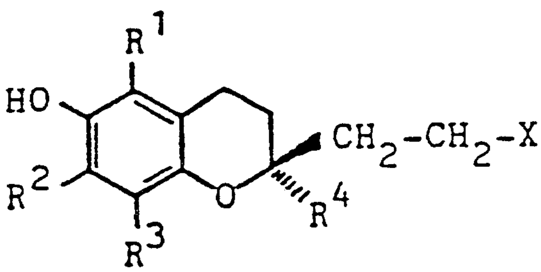

(Ia)

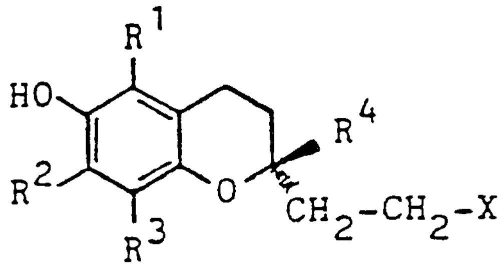

(Ib)

where $R^1$, $R^2$, $R^3$ and $R^4$ are each methyl and X is -OH, Cl, Br, I, $-O-CH_3$, -O-tert.-butyl, -O-acetyl or -O-p-tosyl.

3. (S)-2,5,7,8-tetramethyl-6-hydroxy-2-(2-hydroxy-ethyl)-chroman.

4. (R)-2,5,7,8-tetramethyl-6-hydroxy-2-(2-hydroxy-ethyl)-chroman.

5. (S)-2,5,7,8-tetramethyl-6-hydroxy-2-(2-bromo-ethyl)-chroman.

6. (R)-2,5,7,8-tetramethyl-6-hydroxy-2-(2-bromo-ethyl)-chroman.

7. (S)-2,5,7,8-tetramethyl-6-hydroxy-2-(2-chloro-ethyl)-chroman.

8. (R)-2,5,7,8-tetramethyl-6-hydroxy-2-(2-chloro-ethyl)-chroman.

9. (S)-2,5,7,8-tetramethyl-6-hydroxy-2-(2-acetoxy-ethyl)-chroman.

10. (R)-2,5,7,8-tetramethyl-6-hydroxy-2-(2-acetoxy-ethyl)-chroman.

11. Diastereomeric chromanyl esters of the general formulae IV and IV'

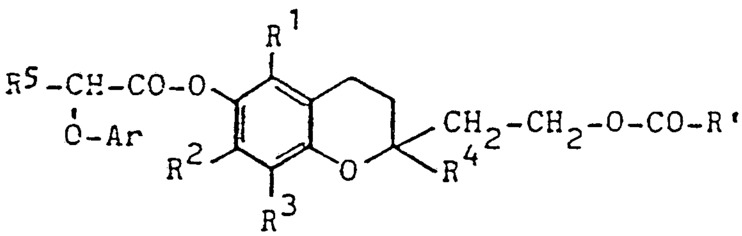

(IV)

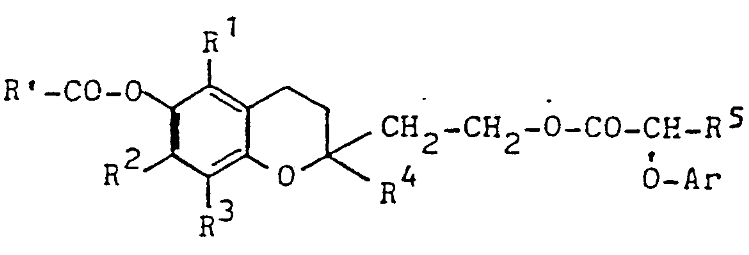

(IV')

where $R^5$ is $C_1$–$C_3$-alkyl, R' is methyl and Ar is phenyl or naphthyl which in turn can be substituted by methyl, chlorine or bromine.

12. 2,5,7,8-Tetramethyl-6-[2'-(o-methyl-p-chlorophenoxy)-propionyloxy]-2-(2-acetoxy-ethyl)-chroman.

13. 2,5,7,8-Tetramethyl-6-acetoxy- 2-[2-(α-naphth-2'-yloxy-propionylethyl]-chroman.

14. 2,5,7,8-Tetramethyl-6-hydroxy-2-[2-(α-naphth-2'-yloxy)-propionylethyl]-chroman.

## Revendications

1. Procédé pour la préparation de nouveaux dérivés de chromane optiquement actifs de formule générales Ia et Ib

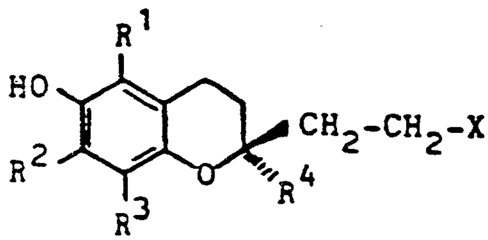

(Ia)

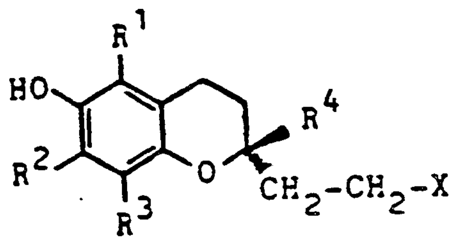

(Ib)

dans lesquelles les radicaux $R^1$ à $R^4$ représentent des atomes d'hydrogène ou des groupes méthyle et X est mis pour un groupement -OH, -O-CO-$R^6$, -O-$R^6$-, O-tosyle, O-mésyle, O-benzène-sulfonyle, un atome de Cl, Br ou I, $R^6$ représentant un groupe alkyle en $C_1$ à $C_4$, caractérisé en ce que

a) on transforme partiellement le racémate I'

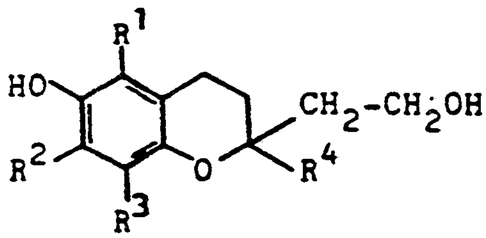

(I')

avec un acide carboxylique de formule II

R'-COOH (II),

dans laquelle R' représente un radical alkyle en $C_1$ à $C_4$, ou avec un ester alkylique inférieur d'un tel acide, en l'ester I"

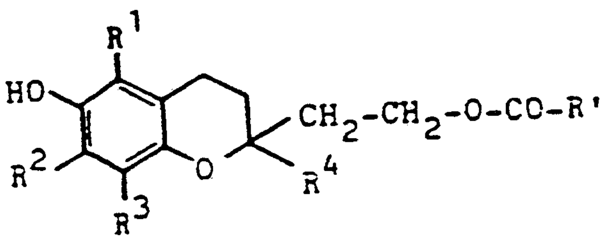

(I")

on acyle celui-ci, avec un halogénure d'acide carboxylique optiquement actif de formule générale III

$R^5$-CH-CO-X (III),
|
O-Ar

dans laquelle $R^5$ représente un groupe méthyle, X représente Cl ou Br et Ar représente un groupe phényle, α-naphtyle ou β-naphtyle qui peut être substitué à son tour par un groupe alkyle en $C_1$ à $C_4$ ou un atome de chlore ou de brome, ou avec l'anhydride d'acide carboxylique correspondant de formule générale V

$R^5$-CH-CO-O-CO-CH-$R^5$ (V),
| |
O-Ar O-Ar

pour obtenir les dérivés de chromane IV

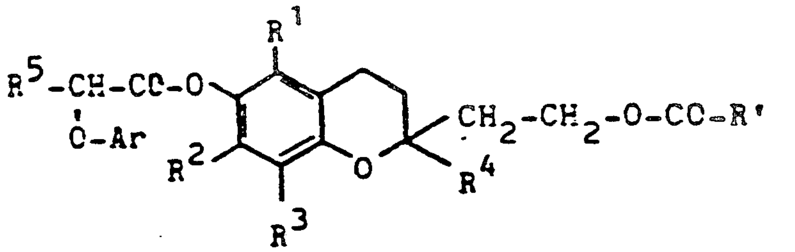

(IV)

on dédouble par cristallisation fractionnée le composé IV se composant de deux esters diastéréoisomères, on hydrolyse les diastéréoisomères purs de la manière habituelle pour obtenir les alcools de formules Ia et Ib respectivement et, le cas échéant, on transforme ceux-ci, de façon connue on soi, en les autres composés de formules Ia et Ib, ou bien

b) on transforme le racémate I′, avec un acide carboxylique III′ ou un ester alkylique inférieur de celui-ci

$R^5$-CH-COOH (III′)
|
O-Ar

en l'ester I‴

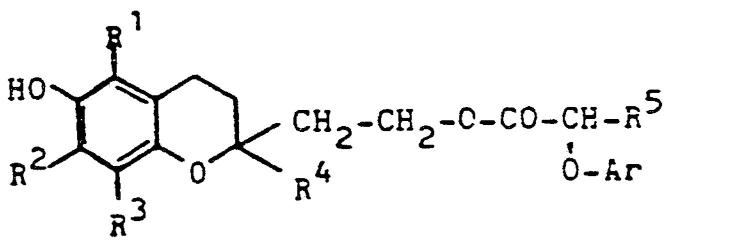

(I‴)

on acyle éventuellement celui-ci avec un halogénure d'acide carboxylique de formule R′-CO-X, dans laquelle X représente Cl, Br ou I, ou avec l'anhydride d'acide carboxylique correspondant pour obtenir IV′

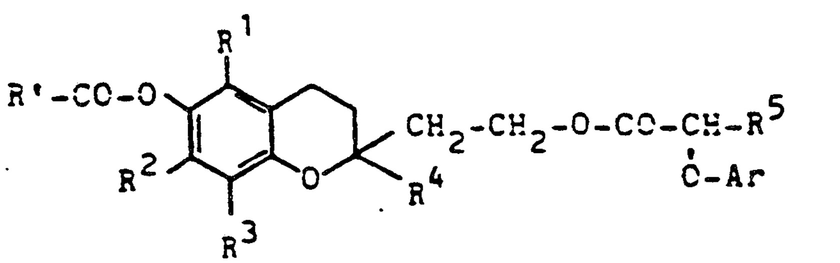

(IV′)

et on dédouble par cristallisation fractionnée le composé I‴ ou IV′ se composant de deux diastéréoisomères, on hydrolyse de la manière habituelle les diastéréoisomères purs pour obtenir les alcools de formules Ia et Ib respectivement et, le cas échéant, on transforme ceux-ci, de façon connue en soi, en les autres composés de formules Ia et Ib.

2. Dérivés de chromane optiquement actifs de formules générales Ia et Ib

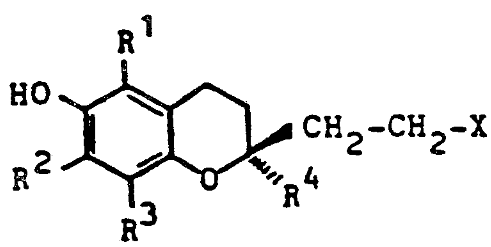

(Ia)

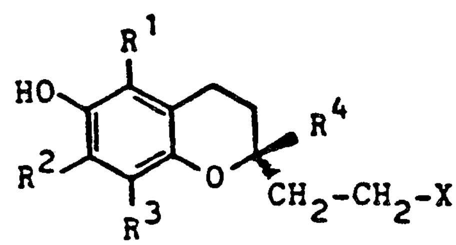

(Ib)

dans lesquelles $R^1$ à $R^4$ sont mis pour des radicaux méthyle et X pour -OH, Cl, Br, I, -O-$CH_3$, un groupement -O-butyle tertiaire, -O-acétyle ou -O-p-tosyle.

3. (S)-2,5,7,8-tétraméthyl-6-hydroxy-2-(2-hydroxy-éthyl)-chromane.

4. (R)-2,5,7,8-tétraméthyl-6-hydroxy-2-(2-hydroxy-éthyl)-chromane.

5. (S)-2,5,7,8-tétraméthyl-6-hydroxy-2-(2-bromo-éthyl)-chromane.

6. (R)-2,5,7,8-tétraméthyl-6-hydroxy-2-(2-bromo-éthyl)-chromane.

7. (S)-2,5,7,8-tétraméthyl-6-hydroxy-2-(2-chloro-éthyl)-chromane.

8. (R)-2,5,7,8-tétraméthyl-6-hydroxy-2-(2-chloro-éthyl)-chromane.

9. (S)-2,5,7,8-tétraméthyl-6-hydroxy-2-(2-acétoxy-éthyl)-chromane.

10. (R)-2,5,7,8-tétraméthyl-6-hydroxy-2-(2-acétoxy-éthyl)-chromane.

11. Esters chromanyliques diastéréoisomères de formules générales IV et IV′

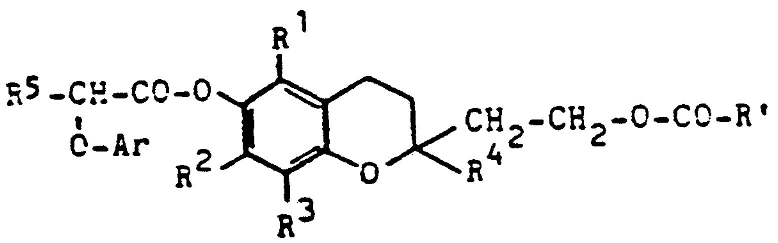

(IV)

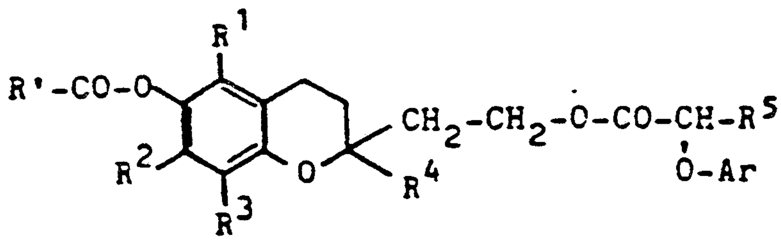

(IV')

dans lesquelles $R^5$ représente un groupe alkyle en $C_1$ à $C_3$, R′ un groupe méthyle et Ar un groupe phényle ou naphtyle qui peut être à son tour substitué par un groupe méthyle, un atome de chlore ou de brome.

12. 2,5,7,8-tétraméthyl-6-[2′-(o-méthyl-p-chloro-phénoxy)-propionyloxy]-2-(2-acétoxy-éthyl)-chromane.

13. 2,5,7,8-tétraméthyl-6-acétoxy-2-[2-(2′-α-naphtoxy)-propionyl-éthyl]-chromane.

14. 2,5,7,8-tétraméthyl-6-hydroxy-2-[2-(2′-α-naphtoxy)-propionyl-éthyl]-chromane.